# EUROPEAN PATENT SPECIFICATION

(11) **EP 2 224 862 B1**
(45) Date of publication and mention of the grant of the patent: **20.06.2018**
(21) Application number: 08866451.1
(22) Date of filing: 18.12.2008
(51) Int. Cl.: A61M 25/04, A61M 25/09, A61B 17/221, A61B 17/22

(54) **SELF EXPANDING WIRE GUIDE**
SELBSTEXPANDIERENDE DRAHTFÜHRUNG
GUIDE DE FIL AUTO-EXTENSIBLE

(30) Priority: 28.12.2007 US 966480
(43) Date of publication of application: 08.09.2010
(73) Proprietor: Cook Medical Technologies LLC, Bloomington, IN 47404 (US)
(72) Inventor: YANG, Xiujiang, Shanghai City 201600 (CN); LU, Wenfeng, Pfafftown, NC 27040 (US)
(74) Representative: Garratt, Peter Douglas
(86) International application number: PCT/US2008/087366
(87) International publication number: WO 2009/085916

(56) References cited:
- WO-A-2007/131100
- US-A1- 2005 165 441
- US-B1- 6 280 450

## Description

### BACKGROUND OF THE INVENTION

During placement of a wire guide, an operator must navigate the wire guide through the body lumen. Often, the body lumen defines a torturous path due to the presence of natural bends and/or curves, or unnatural impediments, such as tumors, build-ups, and/or strictures. The presence of a torturous path may make navigation of a wire guide difficult. For example, the presence of an impediment may block the wire guide from navigating further into the body lumen.

Additionally, wire guide slippage from a target body lumen tends to be a common problem. This often results from the advancement or retraction of other devices over the wire guide. Slippage of the wire guide requires that the placement procedure be repeated, which increases procedure time and potentially causes trauma to the patient.

In view of these current problems, there is an unmet need for a wire guide that can navigate a tortuous path having impediments to a target site and thereafter remain in position at the target site without slipping from the target site. A wire guide as disclosed in the preamble of claim 1 is disclosed in WO 2007/131100.

### SUMMARY OF THE INVENTION

In a first aspect, a wire guide capable of anchoring within a body lumen is provided. An elongate member is provided comprising a first proximal end and a first distal end. An expandable anchoring portion is also provided which is affixed to the first distal end of the elongate member. The expandable anchoring portion comprises a second proximal end and a second distal end and a plurality of expandable members extending between the second proximal end and the second distal end. The expandable anchoring portion self-expands from a collapsed configuration to an expanded configuration. One or more surface features are disposed along the plurality of expandable members to anchor engage the expandable members to one or more walls of the body lumen when the expandable anchoring portion self-expands to the expanded configuration.

In a second aspect, a wire guide system is provided capable of anchoring within a body lumen. The wire guide system comprises an elongate member having a first proximal end and a first distal end. The wire guide system further comprises an expandable anchoring portion affixed to the first distal end of the elongate member. The expandable anchoring portion comprises a plurality of expandable members extending between a second proximal end and a second distal end. The expandable anchoring portion self-expands from a collapsed configuration to an expanded configuration. The wire guide system also comprises a sheathing member disposed over the elongate member. The sheathing member is adapted to retract and resheath relative to the elongate member and the expandable anchoring portion. One or more surface features are disposed along the plurality of expandable members to anchor the expandable members to one or more walls of the body lumen when the expandable anchoring portion self-expands to the expanded configuration.

In a third aspect, which does not form part of the invention, a method of accessing a body lumen is provided. A wire guide system is provided comprising an elongate member having a first proximal end and a first distal end, an expandable anchoring portion affixed to the first distal end of the elongate member, the expandable anchoring portion comprising a plurality of expandable members extending between a second proximal end and a second distal end, the expandable anchoring portion self-expanding from a collapsed configuration to an expanded configuration, and a sheathing member disposed over the elongate member. The sheathing member, being loaded with the elongate member and expandable anchoring portion, is advanced to a target site of the body lumen. The expandable anchoring portion is exposed from within the sheathing member. The expandable anchoring portion thereafter self-expands from the collapsed configuration to the expanded configuration until the expandable anchoring portion engages one or more walls of the body lumen to anchor the expandable anchoring portion within the body lumen.

### BRIEF DESCRIPTION OF THE DRAWINGS

Figure 1 is a side view of a wire guide system;
Figure 2 shows the wire guide system being delivered to a target site within a biliary duct;
Figures 3 and 4 show the sheathing member being retracted to allow the expandable anchoring portion to self-expand and engage the walls of the bilary duct;
Figure 5 shows the sheathing member completely retracted so as to allow the expandable anchoring portion to fully self-expand and anchor against the walls of the body lumen, thereby allowing a stent to be introduced into the biliary duct over the deployed wire guide; and
Figure 6 shows the sheathing member being reintroduced over the elongate member so as to resheath the expandable anchoring portion;
Figure 7 shows an expandable anchoring portion with an elongated distal tip;
Figure 8 shows an expandable anchoring portion with expandable members having serrated edges, the serrated edges being sloped along the distal direction;
Figure 9 shows an expandable anchoring portion having hooks affixed to the expandable members, the hooks having one or more bars disposed therelaong;
Figure 10 shows a sheathing member with a side port thorugh which elongate member is fed into;
Figure 11 shows multiple expandable members having coils disposed about a portion of each of the expandable members;
Figure 12 shows the surfaces of the expandable members covered with barbs;
Figure 13 shows multiple barbs angle outwardly a predetermined amount when expandable anchoring portion is radially expanded;
Figure 14 shows barbs oriented substantially perpendicular to the outer surfaces of expandable members; and
Figure 15 shows the outer surfaces of each of the expandable members comprising surface indentations.

### DETAILED DESCRIPTION OF THE INVENTION

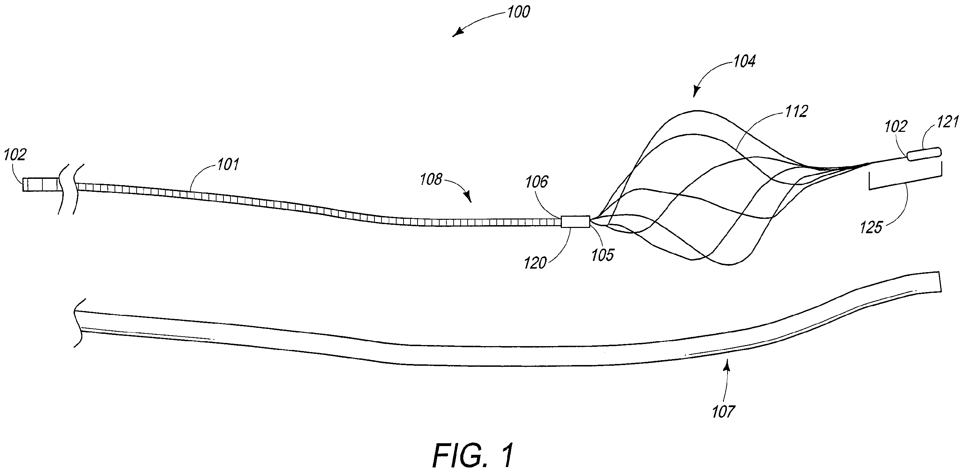

Figure 1 illustrates a self-expanding wire guide system according to a first embodiment of the present invention. The self expanding wire guide system 100 comprises a wire guide 108 and a sheathing member 107. The wire guide 108 comprises an elongate member 101 and an expandable anchoring portion 104. The elongate member 101 has a proximal end 102 and a distal end 116. The elongate member may comprise a relatively tightly wound coil. The expandable anchoring portion 104 as shown in Figure 1 is a self-expandable structure that is adapted to expand from a collapsed configuration so as to engage one or more walls of a body lumen and anchor therewithin. Such anchoring within the body lumen enables access to a particular body lumen for subsequent medical procedures to be performed therewithin, such as in an Endoscopic Retrograde CholangioPancreatography (ERCP) procedure. When expanded, the expandable anchoring portion 104 exerts a radial force that is sufficient to engage one or more walls of the body lumen, thereby reducing the likelihood of slippage from the body lumen. The sheathing member 107 is designed to be disposed over the elongate member 101 and expandable anchoring portion 104 during delivery to the target site. The expandable anchoring portion 104 is in a collapsed configuration when disposed entirely within the sheathing member 107. The sheathing member 107 may be proximally retracted so as to expose the expandable anchoring portion 104, thereby allowing the expandable anchoring portion 104 to self-expand from its collapsed configuration to an expanded configuration. The sheathing member 107 may be further proximally retracted to expose the entire elongate member 101. With the entire elongate member 101 exposed and the expandable anchoring portion 104 anchored within the body lumen, a stabilized pathway is provided over which medical devices (e.g., expandable stent, cannula, or catheter) may be introduced. Such medical devices may be introduced over the elongate member 101 without significant risk of slippage of the elongate member 101 from the body lumen. After the particular procedure is completed, the sheathing member 107 may resheath the elongate member 101 and the expandable anchoring portion 104 and thereafter the wire guide system 100 may be removed from the body lumen.

The elongate member 101 may have a diameter ranging from about .02 inches to about .08 inches. Typical longitudinal lengths of the elongate member may range from about 150 cm to about 450 cm. The exact longitudinal length will depend on the anatomical site being accessed and the type of wire guide exchange being utilized. Any suitable material can be used for the elongate member 101, and a variety of suitable materials are known to those skilled in the art. The material chosen need only be biocompatible and able to be formed into the structures described herein. Examples of suitable materials include stainless steel and nitinol. The elongate member 101 may comprise a wire or a tubular member. Further, the elongate member 12 can be formed of a series of layers, or as a coated core structure. For example, in one embodiment, the elongate member 12 comprises a nitinol core with a PTFE covering.

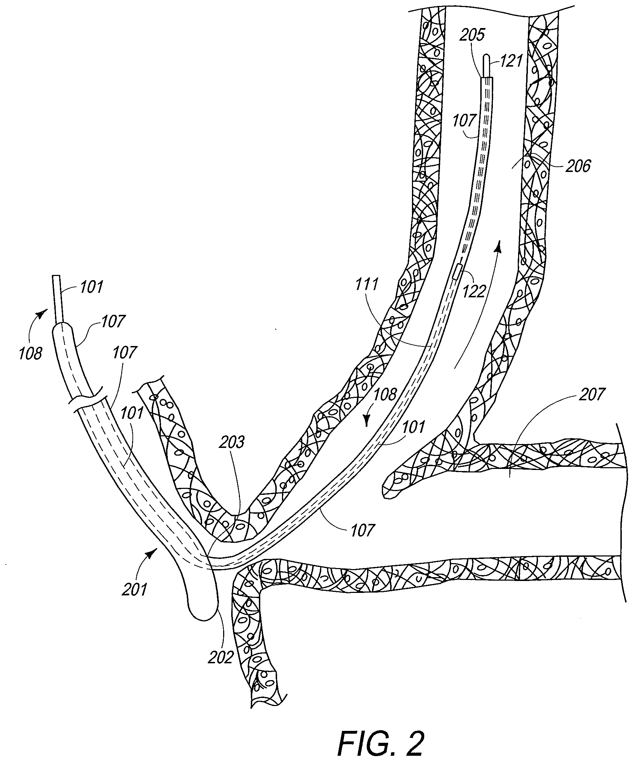

The sheathing member 107 may range from about 4 Fr (1.33 mm) to about 16 Fr. (5.33 mm). Suitable materials for the sheathing member 107 include PTFE, nylon, PU, or any other flexible biocompatible material as known in the art. Preferably, the sheathing member 107 is formed from a polymeric material that possesses flexibility and pushability to navigate around tortuous bends. The sheathing member 107 is slidably moveable relative to the elongate member 101 and expandable anchoring portion 104. During delivery to a target site, the sheathing member 107 is slidably disposed over the elongate member 101 and expandable anchoring portion 104. When reaching the target site, the sheathing member 107 is slidably removed from the expandable anchoring portion 104 and at least a portion of the elongate member 101 to enable the expandable anchoring portion 104 to radially self-expand and engage one or more walls of the body lumen. The sheathing member 107 comprises a wire guide lumen 111 (Figure 2) through which the elongate member 101 and collapsed expandable anchoring portion 104 extends. The wire guide lumen 111 is sized so as to receive the expandable anchoring portion 104 in its collapsed configuration. The sheathing member 107 may also be designed having a first lumen and a second lumen. The first lumen may be configured to receive the proposed self-expanding wire guide 108 (i.e., elongate member 101 and expandable anchoring portion 104 in its collapsed state) or a standard wire guide and the second lumen may be configured for injecting coolant fluid therethrough to deform and cool an expanded expandable anchoring portion 104 to enable resheathing of the sheathing member 107 over the expandable anchoring portion 104, as will be explained in greater detail below. The first lumen may be sized to have a larger inner diameter than the second lumen.

The expandable anchoring portion 104 of Figure 1 is shown in its expanded state. The expandable anchoring portion 104 may comprise a plurality of expandable members 112 to form a basket-like structure in its biased state. Preferably, the expandable anchoring portion 104 may comprise two or three expandable members 112 so as to create a compact structure. The expandable members 112 span between a proximal end 105 and a distal end 106 of the expandable anchoring portion 104. The expandable members 112 extend radially outward from a central longitudinal axis of the elongate member 101. The number of expandable members 112 utilized depends, at least in part, on the radial force necessary to anchor the expandable anchoring portion 104 within a body lumen.

The proximal portion of each of the plurality of expandable members 112 tapers into a proximal cannula 120 which extends circumferentially about the distal end of the elongate member 101 (Figure 1). The distal portion of each of the plurality of expandable members 112 tapers into a distal cannula 121 (Figure 1). The proximal and distal portions of the expandable members 112 may be affixed to their respective cannula 120 and 121 in any number of ways, including welding or soldering. The middle portion of each of the plurality of expandable members 112 radially bows outwards into their biased, expanded configuration. Other means for securing the proximal portion and distal portion of each of the plurality of expandable members 112 is contemplated. For example, the proximal ends of the plurality of expandable members 112 may be soldered directly to the distal end of the elongate member 101, and the distal ends of the plurality of expandable members 112 may be soldered to each other.

The expandable anchoring portion 104 is designed to be moveable from a collapsed configuration to an expanded configuration. The expandable anchoring portion 104 is biased in the expanded configuration. The expandable anchoring portion 104 reverts to the collapsed configuration when disposed within the lumen 111 and constrained by the sheathing member 107. In the collapsed configuration, the expandable members 112 may be substantially parallel to the axis of the elongate member 101. When the sheathing member 107 is proximally retracted so as to expose the expandable members 112 of the expandable anchoring portion 104, the expandable members 112 radially bow outward into their biased state so as to create the basket-like structure shown in Figure 1. Preferably, the expandable members 112 of the expandable anchoring portion 104 are formed from a shape-memory material. Because shape memory materials possess super elastic properties, they can sustain a large deformation at a constant temperature. When the deforming force (i.e., the constraining force provided by the outer sheath 107) is released, they return to their original undeformed shape.

The plurality of expandable members 112 in their expanded state may orient themselves in any number of ways. The embodiment of Figure 1 shows that the plurality of expandable members 112 extend radially outward from a central axis that runs longitudinally along the elongate member 101. Two expandable members 112 are shown extending radially upwards from the central axis of the elongate member 101. Two other expandable members 112 are shown extending radially downward from the central axis of the elongate member 101. Another wire is shows extending radially away from the central axis and out of the plane of the page. Another wire is shown extending radially away from the central axis and into the plane of the page. Alternatively, each of the expandable members 112 may be loosely interwoven with each other. In yet another embodiment, the expandable members 112 may helically extend between the proximal and distal cannulas 120 and 121 to form a braided structure. The expandable anchoring portion 104 may comprise a variety of shapes. For example, the expandable anchoring portion may comprise a spherical shape or a football shape or a dogbone shape. The specific structure of the expandable anchoring portion 104 may depend on numerous factors, including the size of the body lumen that the expandable anchoring portion 104 is to be expanded within and the radial force required to sufficiently anchor the expandable anchoring portion 104 therewithin.

The length of the obstructive member 104 and its expanded diameter varies depending on the particular application. The length may range from about 10 mm to about 50 mm. The expanded diameter may vary from about 3 mm to about 40 mm. The lower ranges of the length and expanded diameter may be suitable for cannulating the biliary tree a small blood vessel and the higher ranges of the length and expanded diameter may be suitable for cannulating the colon. The term "expanded diameter" as used herein refers to the largest separation distance between the plurality of expandable members when the members are in their biased, expanded configuration.

The radial force of the expandable anchoring portion 104 is designed to sufficiently engage one or more walls of body lumen so as to anchor the expandable anchoring portion therewithin. Because the expandable anchoring portion 104 may be engaging healthy tissue, the expandable anchoring portion may be designed to exert a lower radial force than a conventional expandable stent so as to not induce trauma to the healthy tissue. The lower radial force may be achieved by utilizing softer expandable members or fewer expandable members. In a preferred embodiment, the wire diameter of each of the plurality of expandable members 112 is smaller than the elongate member 101 and each of the expandable members 112 is formed from a shape memory material such as nitinol. The smaller wire diameter of the expandable anchoring portion 104 and the shape memory material may in combination help to create an atraumatic expandable anchoring portion 104. The expandable members 112 may also be heat treated to further reduce the stiffness of the expandable members 112 and achieve the desired softness of the expandable members 112. The expandable members 112 may also be coated with a hydrophilic polymer to increase the lubricity of the outer surface of the expandable members 112, thereby softening the expandable members 112 and enhancing the atraumaticness of the expandable anchoring portion 104. The expandable members 112 may be formed from other materials such as stainless steel that has been annealed.

The wire guide 108 is further characterized as having an atraumatic distal tip 125. The distal tip 125 is the region at which the distal end of the expandable members 112 taper down from the expanded region and thereafter converge into the distal cannula 121. The length of the atraumatic distal tip 125 may range from about 5 mm to about 50 mm. The diameter of the distal tip 125 may range from about .2 inches (5.08 mm) to about .05 inches (1.27 mm). The distal tip may be coated with an elastic material having low durometer such as polyether block amide (PEBAX), polyurethane, or silicone to reduce the frictional engagement of the distal tip 125.

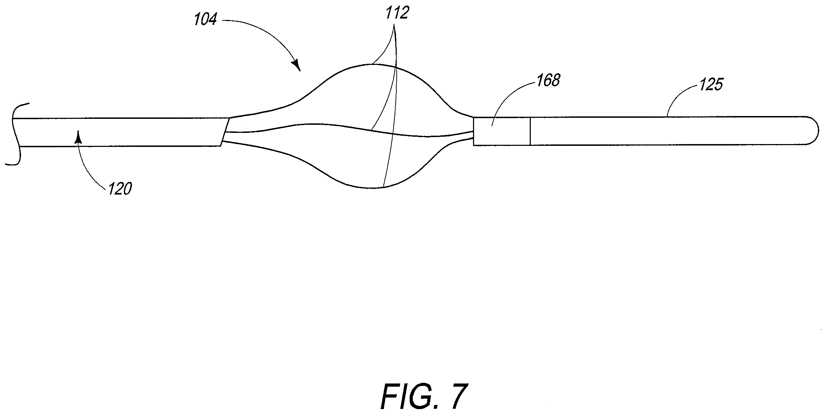

Alternatively, the distal tip 125 may be relatively longer as shown in Figure 7. Figure 7 shows that the distal ends of the expandable members 112 are crimped into an anchoring device 168. The distal end of the anchoring device 168 is affixed to distal tip 125. When preloaded in sheathing member 107, the distal tip 125 may extend pass the distal end of the sheathing member 107. Having such a configuration may facilitate maneuvering through tortuous body lumens with strictures. For example, during advancement of the wire guide system 100 of Figure 1, the sheathing member 107 is pre-loaded with the expandable anchoring portion 104 and elongate member 101 such that the distal end 125 is entirely confined within the sheathing member 107. The body lumen through which the pre-loaded sheathing member 107 attempts to pass through may be narrowed by the strictures to the extent that the outer diameter of the sheathing member 107 may not be able to pass therethrough because of the impediment caused by the strictures. When encountering such a scenario, the wire guide 108 may be advanced out of the sheathing member 107. The wire guide 108 (i.e., elongate member 101 and expandable anchoring portion 104) will be sufficiently small in size to pass through the strictures, and the expandable anchoring portion 104 may not fully expand until it passes the strictures. The longer distal tip 125 provides increased torqueability and pushability through the tortuous body lumen to enhance maneuverability of the wire guide 108 through the tortuous body lumen.

A hydrophilic polymer is preferably coated over the inner surfaces of the sheathing member 107. The hydrophilic polymer preferably has a low coefficient of friction which facilitates resheathing of the sheathing member 107 over the expandable anchoring portion 104. Examples of hydrophilic polymers include polyacrylate, copolymers comprising acrylic acid, polymethacrylate, polyacrylamide, poly(vinyl alcohol), poly(ethylene oxide), poly(ethylene imine), carboxymethylcellulose, methylcellulose, poly(acrylamide sulphonic acid), polyacrylonitrile, poly(vinyl pyrrolidone), agar, dextran, dextrin, carrageenan, xanthan, and guar. The hydrophilic polymers can also include ionizable groups such as acid groups, e.g., carboxylic, sulphonic or nitric groups. The hydrophilic polymers may be cross-linked through a suitable cross-binding compound. The cross-binder actually used depends on the polymer system: If the polymer system is polymerized as a free radical polymerization, a preferred cross-binder comprises 2 or 3 unsaturated double bonds. Alternatively, the lubricious coating may be any biostable hydrogel as is known in the art.

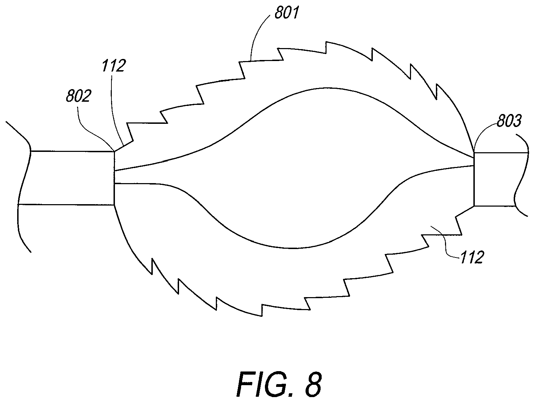

In order to enhance frictional engagement of the expandable members 112 of the expandable anchoring portion 104 with a body lumen, the expandable members 112 may comprise various surface features. For example, Figure 8 shows expandable members 112 having serrated edges 801 extending between the proximal end 803 and the distal end 802 of the expandable anchoring portion 104. The edges 801 are angled in the distal direction such that the expandable anchoring portion 104, even in its expanded state, may be distally maneuvered to the target site. However, because the edges 801are angled or sloped along the distal direction, they substantially prevent movement in the proximal direction, thereby anchoring the expandable anchoring portion 104 within the body lumen. Accordingly, the angled edges 801 provide enhanced anchoring means to the radially self-expanding expandable anchoring portion 104. Preferably, the serrated expandable members 112 are formed from a material (e.g., stainless steel or shape memory alloy) that can sufficiently collapse to fit within the lumen 111 of sheathing member 107.

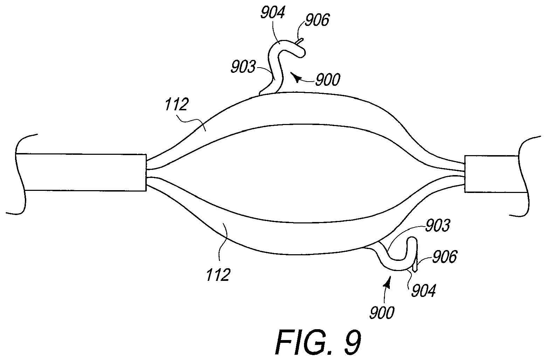

Other means for enhancing the anchoring of the expandable anchoring portion 104 are contemplated. For example, the expandable members 112 may comprise hooks 900 (Figure 9) that engage with the body lumen. The hooks 900 are shown to have a shank 903 and sufficient bend 904 for engaging within the body lumen when the expandable member 112 that the hook is attached to expands. Any number of hooks 900 along each of the expandable members 112 is contemplated. Additionally, the hooks 900 may comprise barbs 906 outwardly projecting from the bends 904 to further help prevent the expandable anchoring portion 104 from slipping out from the body lumen

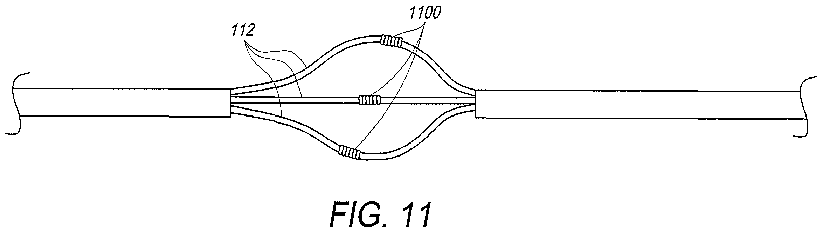

Still alternatively, the expandable members 112 may comprise coils 1100 (Figure 11). The coils 1100 are shown as helically wound about a portion of elongate members 112. The coils 1100 may serve to anchor the expandable anchoring portion 104 within a body lumen. The coils 1100 are preferably flexible coils and made of a radiopaque material (e.g., gold, silver, platinum, tantalum and the like) for use during fluoroscopic visualization. The coils 1100 may allow anchoring of the expandable anchoring portion 103 within the body lumen but yet retain sufficient flexibility to be pushed or pulled through the body lumen without causing trauma to the vasculature or damaging or deforming the expandable members 112. The coils may be positioned at other locations along the expandable members 112.

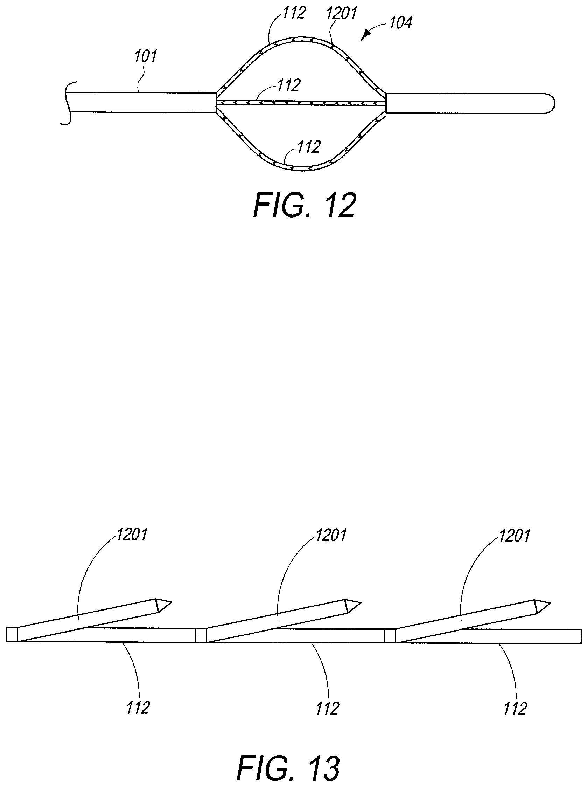

Figure 12 shows another example of expandable members 112 comprising multiple anchors. In particular, the surfaces of the expandable members 112 are shown covered with barbs 1201 that can be formed in the surfaces. The barbs 1201 provide a sandpaper effect of raised, pointed, directional bumps along the surfaces of the expandable members 112. Figure 13 shows a blown-up view of one of the expandable members 112 of Figure 12. Figure 13 shows that the barbs 1201 angle outwardly a predetermined amount when expandable anchoring portion 104 radially expands. Each of the barbs 1201 preferably faces in alignment with a common longitudinal axis of wire guide 108 when the wire guide 108 is in an unexpanded or collapsed configuration. Alternatively, each of the barbs 1201 may be configured adjacent to an outer surface of the expandable members 112 when expandable anchoring portion 104 collapses. In an alternative design, the barbs 1201 may comprise polymeric flaps which reduce trauma to the vasculature. Still alternatively, the expandable members 112 may be formed form a polymeric material in which a portion of each of the outer surfaces expandable members is partially slit to form a flap or bristle that extends outwardly from the outer surfaces.

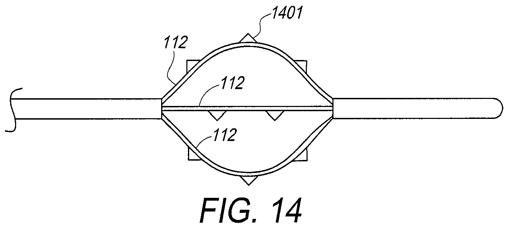

Figure 14 shows another embodiment in which multiple barbs 1401 are formed along the outer surfaces of expandable members 112 so that the barbs 1401 will be directed outwardly when expandable anchoring portion 104 is expanded. The barbs 1401 are shown oriented substantially perpendicular to the outer surfaces of expandable members 112. Such an orientation enables the barbs 1401 to sufficiently grip the vasculature, thereby preventing the likelihood of egress from the target body lumen.

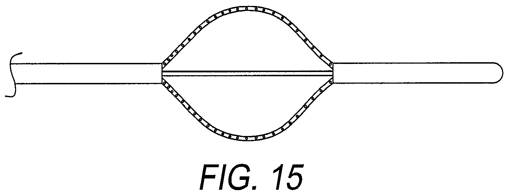

Alternatively, one or more surfaces of the expandable members 112 may comprise a textured surface which provides friction along a surface of the expandable members 12. As an example, surface indentations (e.g., dimples or grooves), as shown in Figure 15, may be utilized to create a textured surface. The textured surface provides surface roughness which may frictionally engage a body lumen. A variety of different shaped surface indentations are contemplated, including spherical, elliptical, rectanguloid. A variety of sized surface indentations are also contemplated. Surface protrusions are also contemplated, such as thin ribbed surfaces. Combinations of the above-described surface features are contemplated. For example, a single expandable member 112 may comprise dimples as well as barbs, polymeric flaps, or other anchoring elements.

It should be recognized that the above-described surface features can be provided in a variety of shapes and configurations other than shown to insure adequate anchoring of the wire guide 108 within a body lumen.

Having described the various components of the wire guide system 100, a method of using the wire guide system 100 will now be described. In particular, a method of cannulating the biliary tree in an ERCP procedure will now be described referring to Figures 2-6. Referring to Figure 2, an endoscope 201 is advanced through the esophagus until it reaches the entrance of the papilla 202. After the endoscope 201 has reached the entrance of the papilla 202, the sheathing member 107 may be inserted into the working channel 203 of the endoscope 201. For purposes of clarity, the proximal portion of the endoscope 201 in Figure 2 is not shown.

Having loaded the sheathing member 107 within the working channel 203, the wire guide 108 is loaded within the sheathing member 107. The expandable members 112 of expandable anchoring portion 104 are constrained by the inner walls of sheathing member 107, thereby collapsing the expandable anchoring portion 104 therewithin. The sheathing member 107 is advanced through the working channel 203 of the endoscope 201 until the distal end 205 of the sheathing member 107 emerges from the distal end of the working channel 203. Figure 2 shows that the distal cannula 121 preferably extends beyond the distal end 205 of sheathing member 107. As the sheathing member 107 emerges from the working channel 203, it is navigated into the biliary duct 206 (Figure 2). The outer diameter of the sheathing member 107 is sufficiently sized so as to be navigated into the biliary duct 206. For cannulation of the biliary duct 206, it is preferable that the distal end of sheathing member 107 is tapered with an outer diameter of about 8 French (2.67 mm) or about 9 french (9 mm).

The distal end 205 of the sheathing member 107 should be positioned sufficiently upstream into the biliary duct 206 such that a sufficient portion of the elongate member 101 is deployed into the bilary duct 206 for subsequent medical devices to be loaded thereon. The medical devices are loaded proximal of the expandable anchoring portion 104 and are not deployed past the anchoring portion 104.

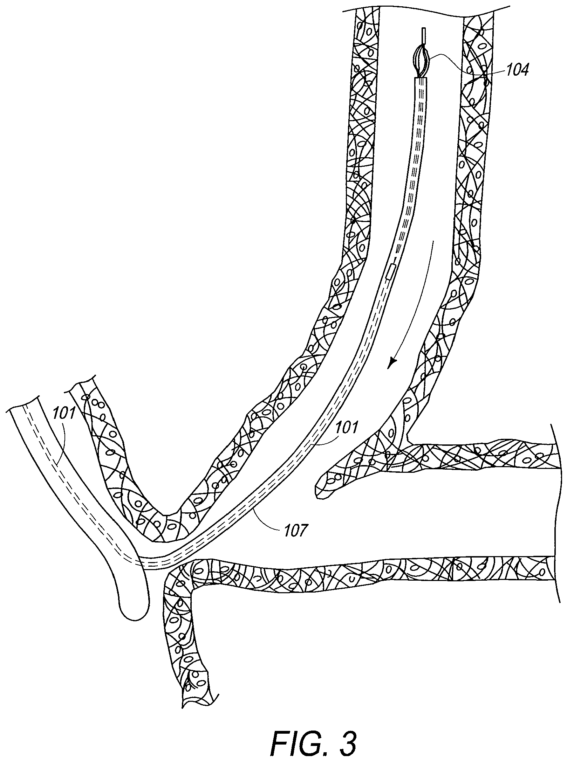

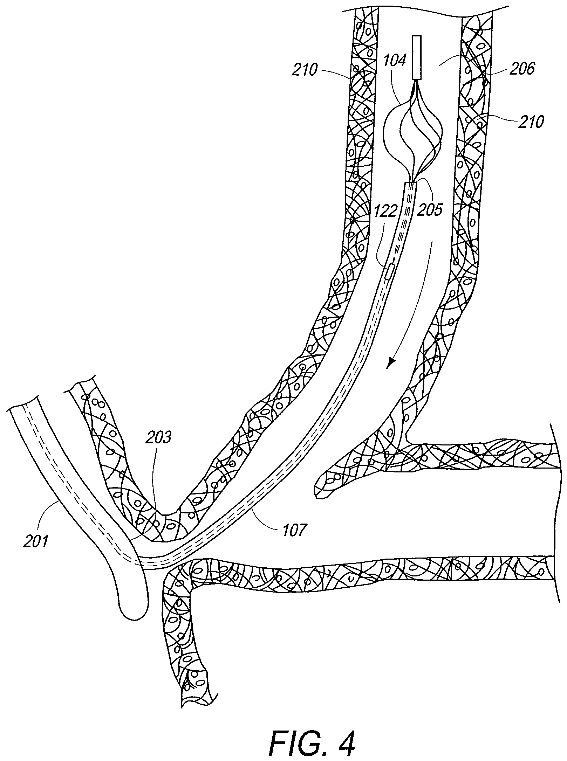

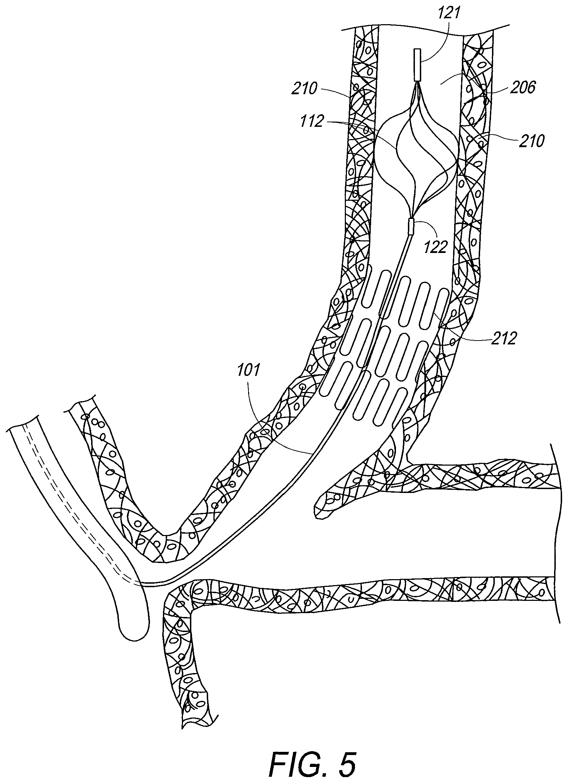

After the distal end 205 of the sheathing member 107 has reached the target site within the bilary duct 206, the proximal end of the sheathing member 107 is proximally retracted to unconstrain a portion of the expandable anchoring portion 104, thereby enabling the expandable anchoring portion 104 to begin radially self-expanding, as shown in Figure 2. Further proximal retraction of the sheathing member 107 (Figure 3) enables a greater portion of the expandable anchoring portion 104 to be exposed and unconstrained, thereby allowing further radial self-expansion of the expandable anchoring portion 104. Still further proximal retraction of the sheathing member 107 (Figure 4) enables the expandable anchoring portion 104 to continue to radially self-expand towards the walls 210 of the body lumen of the bilary duct 206 at the target site. Figure 4 shows that the proximal portion of the expandable anchoring portion 104 and the entire elongate member 101 are constrained within the sheathing member 107. When the distal end 205 of the outer sheath 107 has passed beyond the proximal cannula 122 (Figure 5), the expandable anchoring portion 104 is able to fully radially self-expand and engage the walls 210 of the body lumen, as shown in Figure 5. Figure 5 shows that the expandable members 112 of the expandable anchoring portion 104 have radially bowed outwards to engage against the walls 210 of the body lumen. The expandable members 112 exert a sufficient amount of radial force against the walls 210 to anchor the expandable anchoring portion 104 therewithin, but do not exert excessive radial force so as to cause trauma to the healthy tissue of the walls 210. Various surface features as described in conjunction with Figures 11-15 may be used to impart friction along at least one of the expandable members 112 at a portion of the member 112 which is engaging the walls 210 to reduce the likelihood of egress of the wire guide 108 from the biliary duct 206. Figure 5 shows that the sheathing member 107 has been completely retracted and removed through the working channel 203 of the endoscope 201. Details of separating the sheathing member 107 from the wire guide 108 will be explained below.

It should be noted that elongate member 101 may be moved distally relative to sheathing member 107 to achieve expansion of expandable anchoring portion 104.

A sufficient longitudinal length of the elongate member 101 is exposed for a medical device to now be loaded thereon so that one or more medical devices may now be introduced into the biliary duct 206 along an exposed portion of the elongate member 101. The elongate member 101 remains secured in position within the biliary duct 206 without substantial risk of slippage therefrom because of expandable anchoring portion 104 engaging walls 210 of the body lumen via a variety of surface features disposed along at least a portion of at least one of the expandable members 112 which is in engagement with the walls 210. Figure 5 shows an embodiment in which a stent 212 is introduced through the working channel 203 of the endoscope and along the elongate member 101. The stent 212 is deployed at a location that is proximal of the expandable anchoring portion 104 (Figure 5).

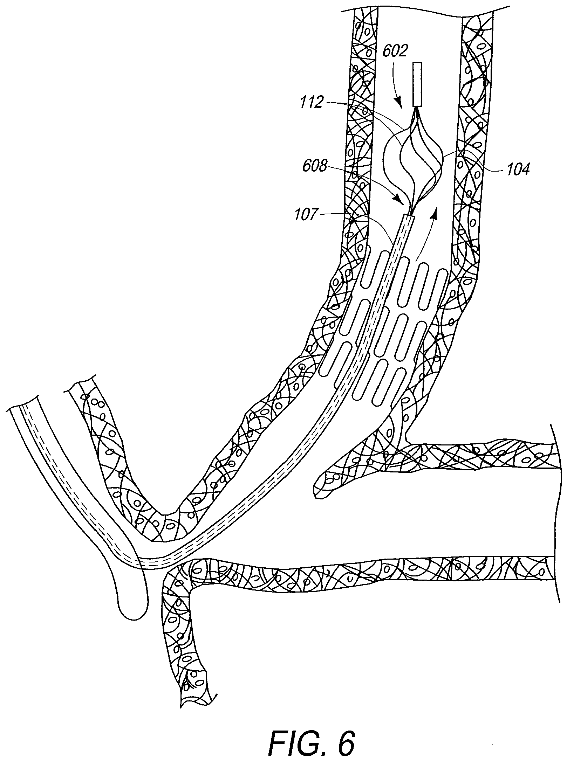

After the particular procedure within the biliary duct 206 has been completed, the elongate member 101 and the expandable anchoring portion 104 may be removed from the bilary duct 206. Figure 6 shows the sheathing member 107 being reintroduced through the working channel of the endoscope and into the bilary duct 206 for the purpose of resheathing over the elongate member 101 and the expanded expandable anchoring portion 104. The tapered regions 601 and 602 (Figure 6) of expanded expandable anchoring portion 104 may be relatively softer than sheathing member 107 to facilitate the resheathing process. Additionally, the resheathing may be facilitated by the coating of a hydrophilic polymer within the inner surfaces of the sheathing member 107. The hydrophilic polymer will reduce the coefficient of friction between the sheathing member 107 and the expandable members 112 thereby allowing the sheathing member 107 to more readily slide over the expandable members 112 and resheath the expandable members 112 into the lumen 111 of the sheathing member 107.

The expandable members 112 may be formed from a shape memory alloy to facilitate collapsing of the member 112 within sheathing member 107. Preferably, the shape memory alloy is a superelastic nickel-titanium alloy, such as nitinol. Nitinol may undergo a substantially reversible phase transformation that allows it to "remember" and return to a previous shape or configuration. The phase transformation may occur between an austenitic phase and a martensitic phase. The phase transformation may be temperature induced in which the expandable members 112 are cooled below its phase transformation temperature (shape memory effect). In one example, a coolant such as saline solution may be injected through the lumen 111 of sheathing member 107. The lumen 111 may be sufficiently sized to allow the saline solution to be injected therethrough. Alternatively, the sheathing member 107 may comprise a first lumen sized for injection of the coolant and a second lumen relatively larger than the first lumen for receiving the elongate member 101 and expandable anchoring portion 104. The saline solution emerges from the distal end of sheathing member 107 and contacts the outer surfaces of the expandable members 112 to cool the nitinol and cause the members 112 to collapse from the expanded configuration.

Additionally and more preferably, the phase transformation may occur by applying stress to the expandable members 112, thereby stress-inducing martensite in what is known as the superelastic effect. In one example utilizing the superelastic effect, stress may applied to nitinol having an initial shape in the austenitic phase to cause a transformation to the martensitic phase without a change in temperature. A return transformation to the austenitic phase may be achieved by removing the applied stress. In general, superelastic alloys are elastic over a wider range than conventional elastic materials such as stainless steel. For example, nitinol can have an elastic range of up to about 8%.

The embodiments as described herein preferably utilize the superelastic properties of nickel-titanium alloys. By virtue of the superelastic properties of such alloys, the expandable members 112 tend to naturally spring back to a larger diameter when a restraining stress is removed. Accordingly, the stress introduced into the expandable members 112 may be released by withdrawing the sheathing member 107 in a proximal direction away from the expandable members 112, whereupon the members 112 expand to its original, expanded configuration by transforming back to the austenitic phase.

After the sheathing member 107 has completely resheathed over the elongate member 101 and over the expandable anchoring portion 104, the sheathing member 107 may be withdrawn from the target site of the biliary duct 206 through the working channel 203 of the endoscope 201.

Wire guide lumen 111 extends from the distal portion of sheathing member 107 to the proximal portion of sheathing member 107. Wire guide lumen 111 preferably has a diameter between about 0.010" (0.25 mm) and about 0.090" (2,29 mm). Elongate member 101 is disposed through wire guide lumen 111 and may exit through the proximal end of sheathing member 107 (Figure 2).

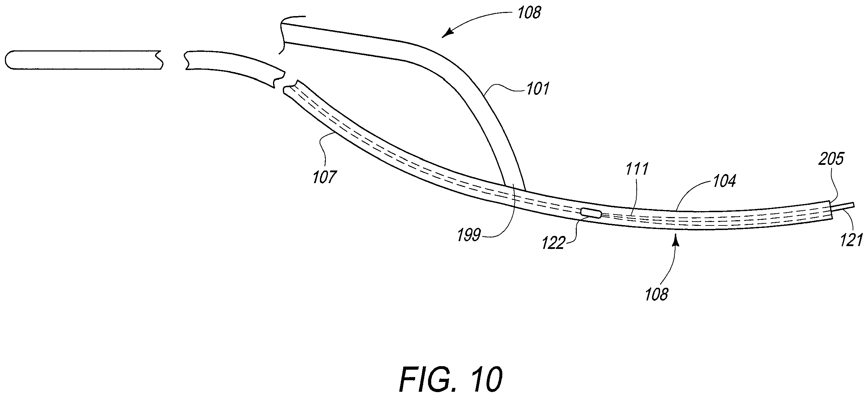

Although the wire guide lumen 111 is shown to extend to the proximal end of the sheathing member 107, the wire guide lumen 111 may also extend to a side port 199 (Figure 10) located along the proximal portion of sheathing member through which the wire guide 108 may be fed. The wire guide 108 extends distally of the port 199. Only the distal end of the wire guide 108 is within the lumen 111, thereby enabling an intermediate wire guide exchange or release of the wire guide 108 from the sheathing member 107. The proximal portion of the wire guide 108 remains outside of the sheathing member 107. Release of the wire guide 108 from the sheathing member 107 may be achieved by pulling the wire guide 108 proximally until the distal portion of wire guide 108 has been removed from the lumen 111. Alternatively, release of the wire guide 108 from the sheathing member 107 may be achieved by pushing the sheathing member 107 distally until side port 199 passes distally beyond the distal end of the wire guide 108. Additional details of these methods, which are referred to as interductal exchanges, are disclosed in U.S. Publication No. 2005-0070794 A1, published on March 31, 2005.

Alternatively, the sheathing member 107 may be separated from the wire guide 108 by withdrawing the sheathing member 107 proximally until it passes over the proximal end of the wire guide 110. Because the devices are not being exchanged over the entire length of the wire guide 108, a short wire guide exchange is possible. Such a short wire guide exchange may decrease surgical procedure time. After separation of the sheathing member 107 from the wire guide 108, other devices may be fed over wire guide 108, which is already inserted at the target site. Alternatively, the wire guide lumen 111 may extend the entire length of the sheathing member 107 to support both short and long wire guide exchanges.

The expandable anchoring portion 104 and sheathing member 107 may comprise radiopaque markers to facilitate visual monitoring during introduction and removal of the elongate member 101 and expandable anchoring portion 104 from a target site.

The wire guide system 100 as described herein eliminates several of the problems encountered by conventional wire guides. For example, slippage of expandable anchoring portion 104 is significantly reduced compared to conventional wire guides that do not possess such a structure. Conventional wire guides typically have a diameter of about 0.035 inches (i.e., about .90 mm) and a typical biliary duct has a lumen size ranging from about 6 mm to about 10 mm. Because the conventional wire guide occupies only about 10% to about 17% of the diameter of the biliary duct and possess only minimal frictional resistance to maintain engagement within the bilary duct, wire guide slippage and loss of cannulation tend to be common problems in several procedures, including ERCP. Loss of cannulation requires repeating navigation of the wire guide into the biliary duct, which is time consuming and may lead to increase trauma and/or injury to the patient. The wire guide system 100 as described herein is able to maintain the elongate member 101 in position because of the self-expandable expandable anchoring portion 104 which engages one or more walls of a body lumen.

Additionally, the problem of wire guide looping is significantly reduced. Wire guide looping may occur when a wire guide is navigated deep into the small intestine or colon. Because the pathway to these areas tend to have several impediments, a conventional wire guide may not be able to negotiate through the curves or the stricture but rather may become caught on the stricture and continue to loop around the stricture. The physician or operator may not be able to visualize the wire guide looping around the stricture/impediment. As a result, the physician or operator continues to unsuccessfully advance the wire guide forward. However, rather than advancing the wire guide forward towards the target site, the wire guide merely continues to loop over the stricture. Even if the looping is detected, the physician or operator has to retract the wire guide and restart the procedure. Generally speaking, such looping normally happens in standard size wire guides or smaller sized wire guides because they are relatively small and flexible. The wire guide system 100 as described herein significantly reduces the risk of looping. The sheathing member 107 is larger in diameter and stiffer than conventional wire guides such that the sheathing member 107 may not be prone to looping around a stricture/impediment.

Because the wire guide 108 is confined within the sheathing member 107 so as to significantly eliminate wire guide looping, the wire guide 108 may deployed without using an endoscope. Radiopaque markers may be selectively affixed to the sheathing member 107 so that the wire guide system 100 can be visually monitored under fluoroscopy during deployment. The ability to advance a wire guide 108 at a target site without an endoscope eliminates the size limitation devices must have as they pass through a working channel of the endoscope. Generally speaking, medical devices such as stents that are fed through a working channel of an endoscope must be small enough to fit through the opening of the working channel, which is normally 10 French (3.33 mm) or smaller. Without use of an endoscope, a stent having a size of about 10 French (3.33 mm) or 20 French (6.66 mm), for example, could be navigated over the wire guide 108.

While preferred embodiments have been described, it should be understood that the preferred embodiments are intended to be limiting in any way, and modifications may be made without departing from the invention. The scope of the invention is defined by the appended claims, and all devices that come within the meaning of the claims, either literally or by equivalence, are intended to be embraced therein. Furthermore, the advantages described above are not necessarily the only advantages of the invention, and it is not necessarily expected that all of the described advantages will be achieved with every embodiment of the invention.

## Claims

1. A wire guide (108) capable of anchoring within a body lumen, comprising:
an elongate member (101)comprising a first proximal end (102) and a first distal end (116),
an expandable anchoring portion (104) affixed to the first distal end of the elongate member, the expandable anchoring portion comprising a second proximal end (105) and a second distal end (106) and a plurality of expandable members (112) extending between the second proximal end and the second distal end, the expandable anchoring portion self-expanding from a collapsed configuration to an expanded configuration; and **characterized by**
one or more surface features (801, 900, 1100, 1201, 1401) disposed along the plurality of expandable members to anchor the expandable members to one or more walls of the body lumen when the expandable anchoring portion self-expands to the expanded configuration.

2. The wire guide of claim 1, wherein the one or more surface features comprises a coil, the coil being helically wound along at least a portion of one of the plurality of expandable members.

3. The wire guide of claim 1, wherein the expandable anchoring portion is tapered at the second proximal end and the second distal end, the expandable anchoring portion biased in the expanded configuration.

4. The wire guide of claim 1, each of the plurality of expandable members comprises a shape memory alloy.

5. The wire guide of claim 1, wherein each of the plurality of expandable members comprises a proximal end and a distal end, the distal end of each of the plurality of expandable members being inserted within a distal cannula, the proximal end of each of the plurality of expandable members being inserted within a proximal cannula, the proximal cannula extending about the first distal end of the elongate member.

6. The wire guide of claim 1, wherein one or more inner surfaces of the sheathing member is coated with a hydrophilic polymer.

7. The wire guide of claim 1, wherein the surface feature comprises a serrated edge, the serrated edge being angled along a distal direction so as to increase frictional contact between the wire and the one or more walls of the body lumen, the serrated edge preventing substantial proximal movement of the expandable anchoring portion.

8. The wire guide of claim 1, wherein the surface feature comprises a hook, the hook further comprising a shank and bend for engaging one or more walls of the body lumen.

9. The wire guide of claim 8, wherein the hook further comprises one or more barbs extending from an outer surface of the hook.

10. The wire guide of claim 1, wherein the surface feature comprises a plurality barbs disposed on at least one of the plurality of expandable surfaces.

11. A wire guide system capable of anchoring within a body lumen,
comprising the wire guide of claim 1; and
a sheathing member disposed over the elongate member, the sheathing member adapted to retract and resheath relative to the elongate member and the expandable anchoring portion.

12. The wire guide system of claim 11, wherein the sheathing member comprises a first lumen and a second lumen.

13. The wire guide system of claim 11, wherein at least one of the plurality of expandable members comprises a radiopaque elements.

14. The wire guide system of claim 11, wherein the one or more surface features comprises a textured surface along an outer surface of at least one of the plurality of expandable members, the textured surface preferably comprising surface indentations or surface protrusions.

## Patentansprüche

1. Drahtführung (108), die innerhalb eines Körperlumens verankert werden kann,
umfassend:
ein langgestrecktes Element (101), das ein erstes proximales Ende (102) und ein erstes distales Ende (116) umfasst,
einen expandierbaren Verankerungsabschnitt (104), der am ersten distalen Ende des langgestreckten Elements befestigt ist, wobei der expandierbare Verankerungsabschnitt ein zweites proximales Ende (105) und ein zweites distales Ende (106) sowie eine Vielzahl von expandierbaren Elementen (112) umfasst, die sich zwischen dem zweiten proximalen Ende und dem zweiten distalen Ende erstrecken, wobei der expandierbare Verankerungsabschnitt aus einer kollabierten Konfiguration in eine expandierte Konfiguration selbstexpandierbar ist; und **gekennzeichnet durch**
ein oder mehrere Oberflächenmerkmale (801, 900, 1100, 1201, 1401), die entlang der Vielzahl von expandierbaren Elementen angeordnet sind, um die expandierbaren Elemente an einer oder mehreren Wänden des Körperlumens zu verankern, wenn der expandierbare Verankerungsabschnitt in die expandierte Konfiguration selbstexpandiert.

2. Drahtführung nach Anspruch 1, wobei das eine oder die mehreren Oberflächenmerkmale eine Spirale umfasst bzw. umfassen, wobei die Spirale spiralförmig entlang mindestens eines Abschnitts eines der Vielzahl von expandierbaren Elementen gewickelt ist.

3. Drahtführung nach Anspruch 1, wobei der expandierbare Verankerungsabschnitt am zweiten proximalen Ende und am zweiten distalen Ende verjüngt ist, wobei der expandierbare Verankerungsabschnitt in die expandierte Konfiguration vorgespannt ist.

4. Drahtführung nach Anspruch 1, wobei jedes der Vielzahl von expandierbaren Elementen eine Formgedächtnislegierung umfasst.

5. Drahtführung nach Anspruch 1, wobei jedes der Vielzahl von expandierbaren Elementen ein proximales Ende und ein distales Ende umfasst, wobei das distale Ende jedes der Vielzahl von expandierbaren Elementen in einer distalen Kanüle eingeführt ist, wobei das proximale Ende jedes der Vielzahl von expandierbaren Elementen in einer proximalen Kanüle eingeführt ist, wobei sich die proximale Kanüle um das erste distale Ende des langgestreckten Elements erstreckt.

6. Drahtführung nach Anspruch 1, wobei eine oder mehrere innere Oberflächen des Umhüllungselements mit einem hydrophilen Polymer beschichtet ist bzw. sind.

7. Drahtführung nach Anspruch 1, wobei das Oberflächenmerkmal einen gezackten Rand umfasst, wobei der gezackte Rand entlang einer distalen Richtung abgewinkelt ist, um den Reibungskontakt zwischen dem Draht und der einen oder der mehreren Wände des Körperlumens zu erhöhen, wobei der gezackte Rand eine erhebliche proximale Bewegung des expandierbaren Verankerungsabschnitts verhindert.

8. Drahtführung nach Anspruch 1, wobei das Oberflächenmerkmal einen Haken umfasst, wobei der Haken ferner einen Schaft und eine Biegung für einen Eingriff in eine oder mehrere Wände des Körperlumens umfasst.

9. Drahtführung nach Anspruch 8, wobei der Haken ferner einen oder mehrere Widerhaken umfasst, der bzw. die sich von einer äußeren Oberfläche des Hakens erstrecken.

10. Drahtführung nach Anspruch 1, wobei das Oberflächenmerkmal eine Vielzahl von Widerhaken umfasst, die auf mindestens einer der Vielzahl von expandierbaren Oberflächen angeordnet sind.

11. Drahtführungssystem, das innerhalb eines Körperlumens verankert werden kann,
umfassend die Drahtführung nach Anspruch 1; und
ein Umhüllungselement, das über dem langgestreckten Element angeordnet ist, wobei das Umhüllungselement bezüglich des langgestreckten Elements und des expandierbaren Verankerungsabschnitts zum Zurückziehen und erneuten Verhüllen ausgelegt ist.

12. Drahtführungssystem nach Anspruch 11, wobei das Umhüllungselement ein erstes Lumen und ein zweites Lumen umfasst.

13. Drahtführungssystem nach Anspruch 11, wobei mindestens eines der Vielzahl von expandierbaren Elementen röntgendichte Elemente umfasst.

14. Drahtführungssystem nach Anspruch 11, wobei das eine oder die mehreren Oberflächenmerkmale eine texturierte Oberfläche entlang einer äußeren Oberfläche mindestens eines der Vielzahl von expandierbaren Elementen umfasst bzw. umfassen, wobei die texturierte Oberfläche vorzugsweise Oberflächenvertiefungen oder Oberflächenvorsprünge umfasst.

## Revendications

1. Guide (108) de fil susceptible de s'ancrer à l'intérieur d'une lumière d'un organisme, comprenant :
un élément allongé (101) comprenant une première extrémité proximale (102) et une première extrémité distale (116),
une partie d'ancrage extensible (104) fixée à la première extrémité distale de l'élément allongé, la partie d'ancrage extensible comprenant une seconde extrémité proximale (105) et une seconde extrémité distale (106) et une pluralité d'éléments extensibles (112) s'étendant entre la seconde extrémité proximale et la seconde extrémité distale, la partie d'ancrage extensible s'auto-étendant depuis une configuration repliée vers une configuration étendue ; et **caractérisée par**
une ou plusieurs caractéristiques de surface (801, 900, 1100, 1201, 1401) disposées le long de la pluralité d'éléments extensibles pour ancrer les éléments extensibles à une ou plusieurs parois de la lumière de l'organisme lorsque la partie d'ancrage extensible s'auto-étend vers la configuration étendue.

2. Guide de fil selon la revendication 1, dans lequel la ou les caractéristiques de surface comprennent une bobine, la bobine étant enroulée en hélice le long d'au moins une partie de l'un des éléments de la pluralité d'éléments extensibles.

3. Guide de fil selon la revendication 1, dans lequel la partie d'ancrage extensible est effilée au niveau de la seconde extrémité proximale et de la seconde extrémité distale, la partie d'ancrage extensible étant sollicitée dans la configuration étendue.

4. Guide de fil selon la revendication 1, chaque élément de la pluralité d'éléments extensibles comprenant un alliage à mémoire de forme.

5. Guide de fil selon la revendication 1, dans lequel chaque élément de la pluralité d'éléments extensibles comprend une extrémité proximale et une extrémité distale, l'extrémité distale de chacun des éléments de la pluralité d'éléments extensibles étant insérée à l'intérieur d'une canule distale, l'extrémité proximale de chacun des éléments de la pluralité d'éléments extensibles étant insérée à l'intérieur d'une canule proximale, la canule proximale s'étendant autour de la première extrémité distale de l'élément allongé.

6. Guide de fil selon la revendication 1, dans lequel une ou plusieurs surfaces internes de l'élément de gainage sont revêtues d'un polymère hydrophile.

7. Guide de fil selon la revendication 1, dans lequel la caractéristique de surface comprend un bord dentelé, le bord dentelé étant incliné le long d'une direction distale de façon à augmenter le contact de friction entre le fil et la ou les parois de la lumière de l'organisme, le bord dentelé empêchant un mouvement proximal important de la partie d'ancrage extensible.

8. Guide de fil selon la revendication 1, dans lequel la caractéristique de surface comprend un crochet, le crochet comprenant en outre une tige et un coude pour une mise en prise avec une ou plusieurs parois de la lumière de l'organisme.

9. Guide de fil selon la revendication 8, dans lequel le crochet comprend en outre un ou plusieurs picots s'étendant depuis une surface externe du crochet.

10. Guide de fil selon la revendication 1, dans lequel la caractéristique de surface comprend une pluralité de picots disposés sur au moins une surface de la pluralité de surfaces extensibles.

11. Système de guide de fil susceptible de s'ancrer à l'intérieur d'une lumière d'organisme, comprenant le guide de fil selon la revendication 1 ; et
un élément de gainage disposé sur l'élément allongé, l'élément de gainage étant conçu pour se rétracter et rengainer par rapport à l'élément allongé et à la partie d'ancrage extensible.

12. Système de guide de fil selon la revendication 11, dans lequel l'élément de gainage comprend une première lumière et une seconde lumière.

13. Système de guide de fil selon la revendication 11, dans lequel au moins un élément de la pluralité d'éléments extensibles comprend des éléments radio-opaques.

14. Système de guide de fil selon la revendication 11, dans lequel la ou les caractéristiques de surface comprennent une surface texturée le long d'une surface externe d'au moins un élément de la pluralité d'éléments extensibles, la surface texturée comprenant de préférence des indentations de surface ou des saillies de surface.
